# EUROPEAN PATENT APPLICATION

(11) **EP 1 245 550 A1**
(43) Date of publication of application: **02.10.2002**
(21) Application number: 00987704.4
(22) Date of filing: 21.12.2000
(51) Int. Cl.: C07B 41/02, C07D 498/18, C07C 69/16, C07C 69/88, C07C 67/28, C07C 67/30, C07C 271/20, C07D 295/12

(54) **NOVEL SELECTIVE DEACETYLATING AGENTS**

(30) Priority: 27.12.1999 JP 37104099
(71) Applicant: FUJISAWA PHARMACEUTICAL CO., LTD., Osaka-shi Osaka 541-8514 (JP)
(72) Inventor: GOTO, Shunsuke, Fujisawa Pharmaceutical Co., Ltd., Osaka-shi, Osaka 541-8514 (JP); YASUDA, Hironobu Fujisawa Pharmaceutical Co., Ltd., Osaka-shi, Osaka 541-8514 (JP); ZANKA, Atsuhiko, Fujisawa Pharmaceutical Co., Ltd., Osaka-shi, Osaka 541-8514 (JP); OMORI, Hiroki, Fujisawa Pharmaceutical Co., Ltd., Osaka-shi, Osaka 541-8514 (JP); HIRABAYASHI, Satoshi Fujisawa Pharma. Co., Ltd., Osaka-shi, Osaka 541-8514 (JP)
(74) Representative: Gille Hrabal Struck Neidlein Prop Roos
(86) International application number: JP0009126
(87) International publication number: WO01047836

(57) **Abstract**

A compound of the formula: 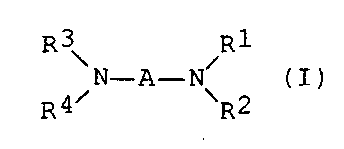 in which R¹ and R² are each lower alkyl or combined together to form lower alkylene, R³ is hydrogen or hydroxy, R⁴ is hydrogen or acyl, and A is lower alkylene, or a salt thereof, which is useful as a selective deacetylating agent.

## Description

### TECHNICAL FIELD

This invention relates to novel compounds having a selective deacetylating action, and a selective deacetylating method using the compounds. More particularly, this invention relates to novel hydroxylamine compounds or amine compounds having a selective deacetylating action, to a selective deacetylating method using the compounds, particularly a selective deacetylating method for the production of compounds useful as materials for medicaments.

### BACKGROUND ART

Conventionally, the compound indicated below is known as a deacetylating agent for p-nitrophenyl acetate (J. Amer. Chem. Soc., 90, 5883 (1969)). However, the deacetylating method using this agent is conducted in a very diluted condition, and the yield in the synthesis of this known compound is low. For this reason, the deacetylating method using the compound indicated below as a reagent is not a convenient method. In addition, the above reference document does not mention the selective deacetylating action of this invention at all.

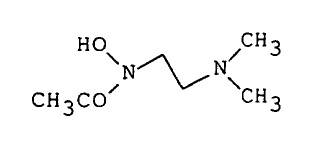

The inventors of this invention earnestly conducted research to obtain selective deacetylating agents being convenient, highly selective and high in yield, and have found selective deacetylating compounds of this invention and a selective deacetylating method using the compounds, thereby completing this invention.

### DISCLOSURE OF INVENTION

The compounds for use as the selective deacetylating compounds of this invention are indicated below: 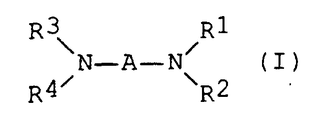 in which R¹ and R² are each lower alkyl or combined together to form lower alkylene, R³ is hydrogen or hydroxy, R⁴ is hydrogen or acyl, and A is lower alkylene, or salts thereof.

Furthermore, among these compounds, a novel compound is a compound indicated below: 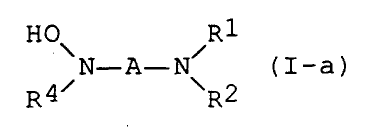 in which R¹ and R² are each lower alkyl or combined together to form lower alkylene, R⁴ is acyl, and A is lower alkylene; however, when R¹ and R² are each lower alkyl, R⁴ is acyl other than lower alkanoyl, or a salt thereof.

Suitable salts of the object compound (I) may include a salt with an acid, such as an inorganic acid addition salt (e.g. hydrochloride, hydrobromide, sulfate, phosphate, etc.), an organic acid addition salt (e.g. formate, acetate, trifluoroacetate, maleate, tartrate, fumarate, methanesulfonate, benzenesulfonate, etc.);
a salt with an acidic amino acid (e.g. arginine, aspartic acid, glutamic acid, etc.); and the like.

In this specification, suitable examples and illustrations of the various definitions that the present invention includes within the scope thereof are explained in detail as follows, but the definitions are not limited to these.

The term "lower" is intended to mean 1 to 6 carbon atoms, preferably 1 to 4 carbon atoms, unless otherwise indicated.

Suitable "lower alkyl" may include straight or branched one, such as methyl, ethyl, propyl, isopropyl, butyl, tert-butyl, pentyl, hexyl, and the like, in which the most preferable one may be methyl, ethyl, and the like for R¹ and/or R².

Suitable "lower alkylene" may include straight or branched one, such as methylene, ethylene, trimethylene, tetramethylene, pentamethylene, hexamethylene, methylmethylene, ethylethylene, propylene, and the like, in which the most preferable one may be ethylene, trimethylene, and the like for A, and pentamethylene for a group formed by R¹ and R².

Suitable "acyl" may include carbamoyl; aliphatic acyl; and acyl including an aromatic ring and referred to as aromatic acyl, and acyl including a heterocyclic ring and referred to as heterocyclic acyl.

Suitable examples of the acyl group may be as follows:
carbamoyl; thiocarbamoyl; sulfamoyl;
aliphatic acyl, such as lower alkanoyl (e.g. formyl, acetyl, propanoyl, butanoyl, 2-methylpropanoyl, pentanoyl, 2,2-dimethylpropanoyl, hexanoyl, etc.);
lower alkoxycarbonyl (e.g. methoxycarbonyl, ethoxycarbonyl, t-butoxycarbonyl, t-pentyloxycarbonyl, hexyloxycarbonyl, etc.);
lower alkylsulfonyl (e.g. methylsulfonyl, ethylsulfonyl, etc.);
lower alkoxysulfonyl (e.g. methoxysulfonyl, ethoxysulfonyl, etc.);
mono(or di or tri)-halo(lower)alkylsulfonyl (e.g. fluoromethylsulfonyl, dichloromethylsulfonyl, trifluoromethylsulfonyl, chloromethylsulfonyl, dichloromethylsulfonyl, trichloromethylsulfonyl, 1 or 2-fluoroethylsulfonyl, 1 or 2- chloroethylsulfonyl, etc.) or the like.

Aromatic acyl, such as aroyl (e.g. benzoyl, toluoyl, naphthoyl, etc.);
ar(lower)alkanoyl [e.g. phenyl(lower)alkanoyl (e.g. phenylacetyl, phenylpropanoyl, phenylbutanoyl, phenylisobutanoyl, phenylpentanoyl, phenylhexanoyl, etc.), naphthyl(lower)alkanoyl (e.g. naphthylacetyl, naphthylpropanoyl, naphthylbutanoyl, etc.), etc.];
ar(lower)alkenoyl [e.g. phenyl(lower)alkenoyl (e.g. phenylpropenoyl, phenylbutenoyl, phenylmethacryloyl, phenylpentenoyl, phenylhexenoyl, etc.), naphthyl(lower)alkenoyl (e.g. naphthylpropenoyl, naphthylbutenoyl, etc.), etc.];
ar(lower)alkoxycarbonyl [e.g. phenyl(lower)alkoxycarbonyl(e.g. benzyloxycarbonyl, etc.), etc.];
aryloxycarbonyl (e.g. phenoxycarbonyl, naphthyloxycarbonyl, etc.);
aryloxy(lower)alkanoyl (e.g. phenoxyacetyl, phenoxypropionyl, etc.);
arylglyoxyloyl (e.g. phenylglyoxyloyl, naphthylglyoxyloyl, etc.);
arylsulfonyl (e.g. phenylsulfonyl, p-tolylsulfonyl, etc.); and the like.

Heterocyclic aryl, such as:
heterocycliccarbonyl;
heterocyclic(lower)alkanoyl (e.g. heterocyclicacetyl, heterocyclicpropanoyl, heterocyclicbutanoyl, heterocyclicpentanoyl, heterocyclichexanoyl, etc.);
heterocyclic(lower)alkenoyl (e.g. heterocyclicpropenoyl, heterocyclicbutenoyl, heterocyclicpentenoyl, heterocyclichexenoyl, etc.);
heterocyclicglyoxyloyl; and the like.

Suitable "heterocyclic moiety" in the terms of the above-mentioned "heterocyclic carbonyl," "heterocyclic(lower)alkyl," "heterocyclic(lower)alkenoyl" and "heterocyclicglyoxyloyl" means, more particularly, a saturated or unsaturated, monocylic or polycyclic heterocyclic group containing at least one hetero-atom, such as an oxygen, sulfur, nitrogen atom, and the like.

Especially preferable heterocylic groups may be:
unsaturated 3 to 8-membered (more preferably 5 or 6-membered) heteromonocyclic group containing 1 to 4 nitrogen atom(s), for example, pyrrolyl, pyrrolinyl, imidazolyl, pyrazolyl, pyridyl, dihydropyridyl, pyrimidyl, pyrazinyl, pyridazinyl, triazolyl (e.g. 4H-1,2,4-triazolyl, 1H-1,2,3-triazolyl, 2H-1,2,3-triazoly, etc.), tetrazolyl(e.g. 1H-tetrazolyl, 2H-tetrazolyl, etc.), etc.;
saturated 3 to 8-membered (more preferably 5 or 6-membered) heteromonocyclic group containing 1 to 4 nitrogen atom(s), for example, pyrrolidinyl, imidazolidinyl, piperidyl, piperazinyl, etc.;
unsaturated condensed 7 to 12-membered (more preferably 9 or 10-membered) heterocyclic group containing 1 to 4 nitrogen atom(s), for example, indolyl, isoindolyl, indolinyl, indolizinyl, benzimadazolyl, quinolyl, isoquinolyl, indazolyl, benzotriazolyl, etc.;
unsaturated 3 to 8-membered (more preferably 5 or 6-membered) heteromonocyclic group containing 1 to 2 oxygen atom(s) and 1 to 3 nitrogen atom(s), for example, oxazolyl, isooxazolyl, oxadiazolyl (e.g. 1,2,4-oxadiazolyl, 1,3,4-oxadiazolyl, 1,2,5-oxadiazolyl, etc.) etc.;
saturated 3 to 8-membered (more preferably 5 or 6-membered) heteromonocyclic group containing 1 to 2 oxygen atom(s) and 1 to 3 nitrogen atom(s), for example, oxazolidinyl, morphorinyl, sydnonyl, etc.;
unsaturared condensed 7 to 12-membered (more preferably 9 or 10-membered) heterocyclic group containing 1 to 2 oxygen atom(s) and 1 to 3 nitrogen atom(s), for example, benzoxazolyl, benzoxadiazolyl, etc.;
unsaturated 3 to 8-membered (more preferably 5 or 6-membered) heteromonocyclic group containing 1 to 2 sulfur atom(s) and 1 to 3 nitrogen atom(s), for example, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc.); dihydrothiazinyl,etc.;
saturated 3 to 8-membered (more preferably 5 or 6-membered) heteromonocyclic group containing 1 to 2 sulfur atom(s) and 1 to 3 nitrogen atom(s), for example, thiazolidinyl, etc.;
unsturated 3 to 8-membered (more preferably 5 or 6-membered) heteromonocyclic group containing 1 to 2 sulfur atom(s), for example, thienyl, dihydrodithiinyl, dihydrodithionyl, etc.;
unsaturated condensed 7 to 12-membered (more preferably 9 or 10-membered) heterocyclic group containing 1 to 2 sulfur atom(s) and 1 to 3 nitrogen atom(s), for example, benzothiazolyl, benzothiadiazolyl, etc.;
unsaturated 3 to 8-membered (more preferably 5 or 6-memberd) heteromonocyclic group containing an oxygen atom, for example, furyl, etc.;
unsaturated 3 to 8-membered (more preferably 5 or 6-membered) heteromonocyclic group containing an oxygen atom and 1 to 2 sulfur atom(s), for example, dihydrooxathiinyl, etc.;
unsaturated condensed 7 to 12-membered (more preferably 9 or 10-membered) heterocyclic group containing 1 to 2 sulfur atom(s), for example, benzothienyl, benzodithiinyl, etc.;
unsaturated condensed 7 to 12-membered (more preferably 9 or 10-membered) heterocyclic group containing an oxygen atom and 1 to 2 sulfur atom(s), for example, benzoxathiinyl; and the like.

The above-mentioned acyl moiety may have one or more (preferably one to three) identical or different suitable substituents, for example, the above-mentioned lower alkyl; the undermentioned lower alkoxy; lower alkylthio having the above-mentioned lower alkyl moiety; lower alkylamino having the above-mentioned lower alkyl moiety; the undermentioned cyclo(lower)alkyl; the undermentioned cyclo(lower)alkenyl; halogen; amino; the undermentioned protected amino; hydroxy; the undermentioned protected hydroxy; cyano; nitro; carboxy; the undermentioned protected carboxy; sulfo; sulfamoyl; imino; oxo; amino(lower)alkyl having the above-mentioned lower alkyl moiety; carbamoyloxy; hydroxy(lower)alkyl having the above-mentioned lower alkyl moiety; diamino(lower)alkyliden having the undermentioned lower alkyliden; di(lower)alkylamino having the above-mentioned lower alkyl moiety; di(lower)alkylamino(lower)alkyl having the above-mentioned lower alkyl moiety; heterocyclic(lower)alkyl having the above-mentioned heterocyclic moiety and lower alkyl moiety, and the like.

Preferable examples of acyl defined as described above may be lower alkanoyl, lower alkoxycarbonyl, C₆-C₁₀ aroyl, and the like, in which the most preferable example may be acetyl, propionyl, isopropoxycarbonyl, tert-butoxycarbonyl, benzoyl, or the like.

Preferable examples of "acyl," other than "lower alkanoyl," may be the above-mentioned acyl excluding "lower alkanoyl," in which more preferable one may be lower alkoxycarbonyl, C₆-C₁₀ aroyl, and the like, and particularly preferable one may be isopropoxycarbonyl, tert-butoxycarbonyl, benzoyl, and the like.

Suitable "lower alkoxy" may be a straight or branched one, such as methoxy, ethoxy, propoxy, isopropoxy, butoxy, tert-butoxy, pentyloxy, hexyloxy, and the like.

Suitable "cyclo(lower)alkyl" may be cyclo(C₃-C₆)alkyl, such as cyclopropyl, cyclobutyl, cyclopentyl, cyclohexyl, and the like.

Suitable "cyclo(lower)alkenyl" may be cyclo(C₃-C₆)alkenyl, such as cyclopropenyl, cyclobutenyl, cyclopentenyl, cyclohexenyl, and the like.

Suitable "protected amino" may be a common protected amino group protected by the above-mentioned acyl, and the like.

Suitable "protected hydroxy " may include common protected hydroxy, such as substituted or unsubstituted arylmethyloxy (e.g. benzyl, lower alkoxybenzyl, etc.), acyloxy, substituted silyloxy (e.g. tert-butyldiphenylsilyl, etc.), and the like, in which preferable examples may be C₆-C₁₀ arylmethyloxy, lower alkoxycarbonyloxy, C₆-C₁₀ arylcarbonyloxy, and the like, and the most preferable example may be benzyloxy, isopropoxycarbonyloxy, tert-butoxycarbonyloxy, benzoyloxy, or the like.

Suitable "lower alkanoyl" may include formyl, acetyl, propanoyl, butanoyl, 2-methylpropanoyl, pentanoyl, 2,2-dimethylpropanoyl, hexanoyl, and the like, in which more preferable one may be C₁-C₄ alkanoyl, and the like, and the most preferable one may be acetyl, propionyl, or the like.

"Esterified carboxy" may include the following.

Suitable example of the ester moiety of an esterified carboxy may be the ones, such as lower alkyl ester (e.g. methyl ester, ethyl ester, propyl ester, isopropyl ester, butyl ester, isobutyl ester, tert-butyl ester, pentyl ester, hexyl ester, etc.), for example, lower alkanoyloxy(lower)alkyl ester [e.g. acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(or 2- or 3- or 4-)acetoxybutyl ester, 1-(or 2-)propionyloxyethyl ester, 1-(or 2- or 3-)propionyloxypropyl ester, 1-(or 2-)butyryloxyethyl ester, 1-(or 2-)isobutyryloxyethyl ester, (1- or 2-)pivaloyloxyethyl ester, 1-(or 2-)hexanoyloxyethyl ester, isobutyryloxymethyl ester, 2-ethylbutyryloxymethyl ester, 3,3-dimethylbutyryloxymethyl ester, 1-(or 2-)pentanoyloxyethyl ester, etc.], aroyl(lower)alkyl ester, for example, benzoyl(lower)alkyl ester (e.g. phenacyl ester, etc.), lower alkane sulfonyl(lower)alkyl ester (e.g. 2-mesylethyl ester, etc.), mono(or di or tri)-halo(lower)alkyl ester (e.g. 2-iodoethyl ester, 2,2,2-trichloroethyl ester, etc.);
lower alkoxycarbonyloxy(lower)alkyl ester [e.g. methoxycarbonyloxymethyl ester, ethoxycarbonyloxymethyl ester, propoxycarbonyloxymethyl ester, tert-butoxycarbonyloxymethyl ester, 1-(or 2-)methoxycarbonyloxyethyl ester, 1-(or 2-)ethoxycarbonyloxyethyl ester, 1-(or 2-isopropoxycarbonyloxyethyl ester, etc.], phthalidylidene(lower)alkyl ester, or (5-lower alkyl-2-oxo-1,3-dioxol-4-yl) (lower)alkyl ester [e.g. (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester, etc.]; lower alkenyl ester (e.g. vinyl ester, allyl ester, etc.); lower alkynyl ester (e.g. ethynyl ester, propynyl ester, etc.); ar(lower)alkyl ester which may have at least one suitable substituent(s) (e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester, etc.); aryl ester which may have at least one suitable substituent(s) (e.g. phenyl ester, 4-chlorophenyl ester, tolyl ester, tert-butylphenyl ester, xylyl ester, mesityl ester, cumenyl ester, etc.); phthalidyl ester; and the like.

Among them, preferable example may be lower alkyl ester, and the most preferable example may be ethyl ester

Suitable "lower alkylidene" may include straight or branched one, such as methylidene, ethylidene, propylidene, isopropylidene, butylidene, pentylidene, hexylidene, methyl methylidene, ethyl methylidene, propylidene, and the like.

Suitable "aroyl" may include C₆-C₁₀ aroyl, such as benzoyl, toluoyl, naphthoyl, etc.

In this invention, for example, as described in the undermentioned reactions, deacetylation can be carried out selectively when acetyl and esterified carboxy groups are present or even when identical acetyl groups are present if they have reactivity slightly different from each other. 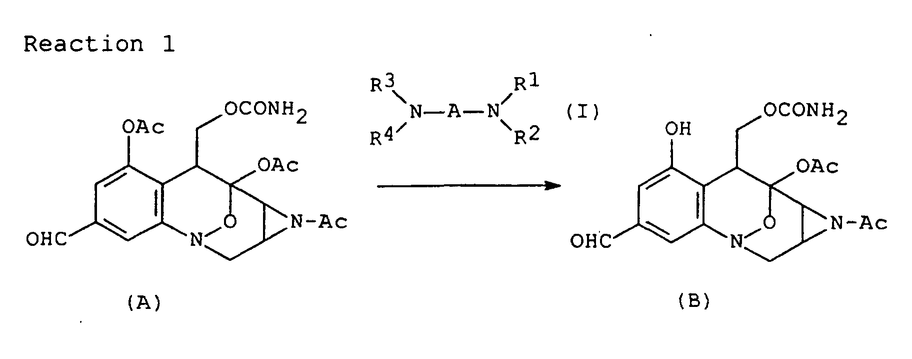 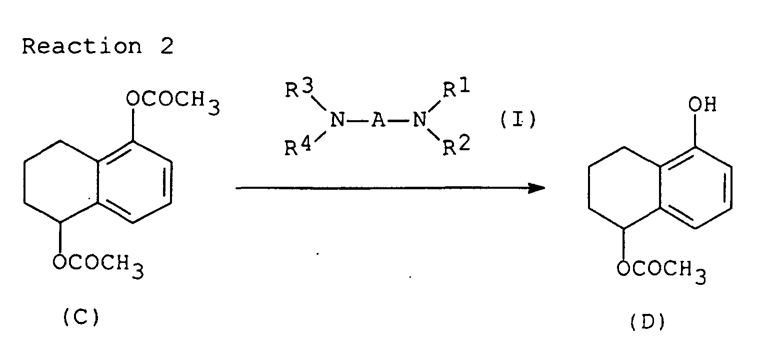 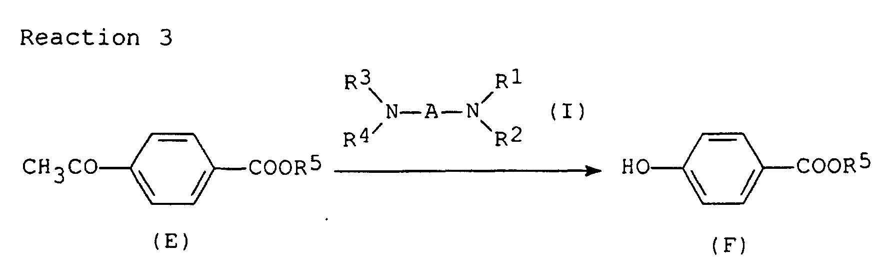 in which R¹ and R² are each lower alkyl or combined together to form lower alkylene, R³ is hydrogen or hydroxy, R⁴ is hydrogen or acyl, -COOR⁵ is esterified carboxy, A is lower alkylene, and Ac is acetyl.

These reactions can be carried out by reacting a raw material compound with an appropriate amount of the compound (I) in an appropriate temperature range under from ambient temperature to warming in a solvent which does not adversely affect the reaction, such as methanol, tetrahydrofuran, etc.

Compounds (A) and (B) are known in Japanese Laid-open Patent Application No. Sho 61-10590 for example, and known to have an antitumor activity. In addition, the Publication describes a solvolysis reaction using sodium bicarbonate from compound (A) to compound (B). However, the yield of the reaction is approximately 55%. It is thus difficult to say that the method is an efficient synthesis method.

Furthermore, the Publication describes synthesis from compound (B) to compound (G) as indicated below. The compound (G) is used for clinical tests as an antitumor agent at present.

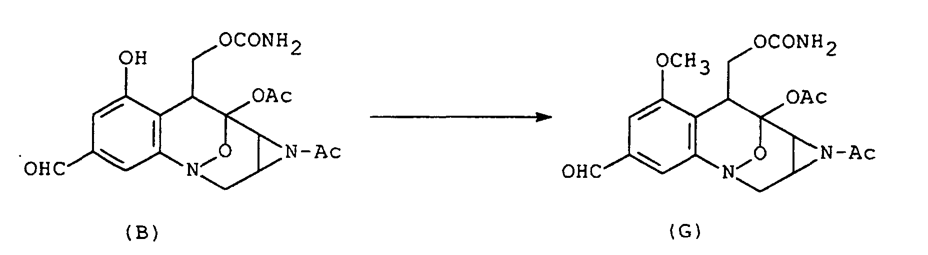

Still further, the compound (A) and the like have one or more asymmetric centers and can be present as an enantiomer or a diastereomer. In both cases, it is needless to say that the compound (A) and the like can be used as a starting compound for the selective deacetylation reaction.

Specific examples of the compounds usable as the selective deacetylating agent of this invention are enumerated below. However, the compounds are not limited to these. 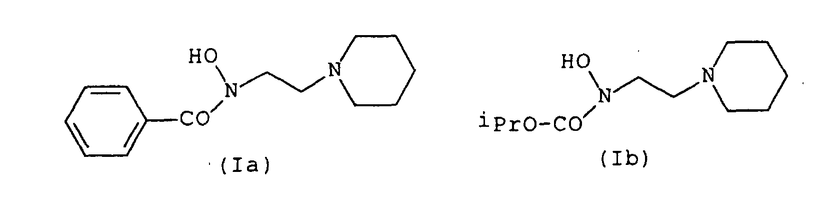 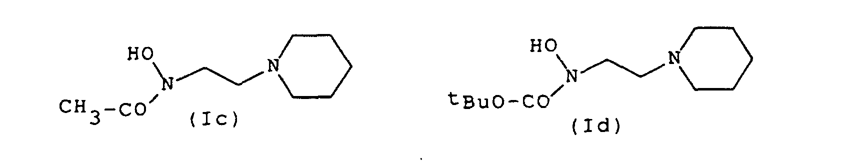 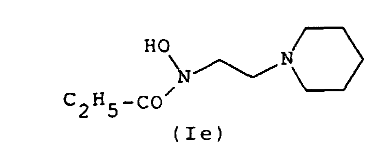 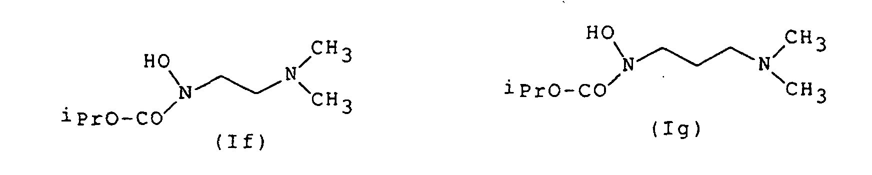 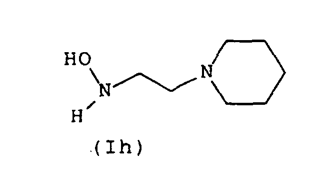 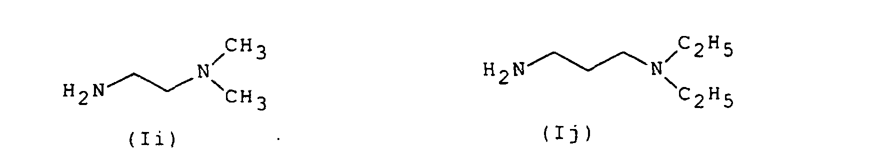 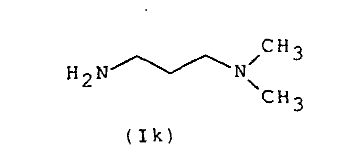 in which iPr is isopropyl and tBu is tert-butyl.

Among these compounds (Ia) to (Ik), it is preferable that the compounds (Ia) and (Ib) are used for selective deacetylation because of ease of handling (crystalinity, stability, etc.).

These compounds can be synthesized by the production method indicated below, the methods described in the undermentioned preparations, or methods similar thereto. However, the method for synthesis is not limited to these methods.

### Production method

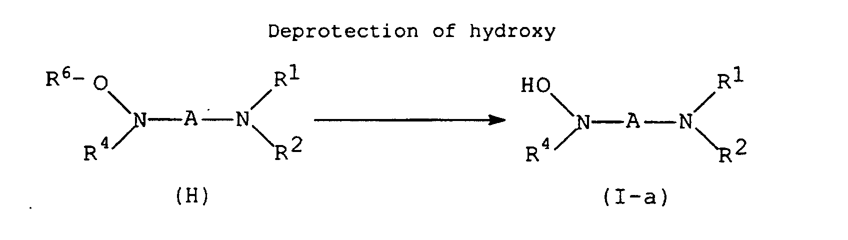

in which R¹, R², R³ and R⁴ are each as defined above, and R⁶ is a hydroxy protective group.

### Explanation of production method

The compound (I-a) can be obtained by subjecting the compound (H) to the elimination reaction of the hydroxy protective group.

This reaction is usually carried out in accordance with a conventional method, such as solvolysis/hydrolysis, reduction, etc.

### (i) Solvolysis/hydrolysis

Solvolysis/hydrolysis is preferably carried out in the presence of a base or an acid. Suitable base may include an alkali metal hydroxide (e.g. sodium hydroxide, potassium hydroxide, etc.), alkali earth metal hydroxide (e.g. magnesium hydroxide, calcium hydroxide, etc.), alkali metal hydride (e.g. sodium hydride, potassium hydride, etc.), alkali earth metal hydride (e.g. calcium hydride, etc.), alkali metal alkoxide (e.g. sodium methoxide, sodium ethoxide, potassium t-butoxide, etc.), alkali metal carbonate (e.g. sodium carbonate, potassium carbonate, etc.), alkali earth metal carbonate (e.g. magnesium carbonate, calcium carbonate, etc.), alkali metal bicarbonate (e.g. sodium bicarbonate, potassium bicarbonate etc.), and the like.

Suitable acid may include an organic acid (e.g. formic acid, acetic acid, propionic acid, trichloroacetic acid, benzene sulfonic acid, p-toluenesulfonic acid, etc.) and an inorganic acid (e.g. hydrochloric acid, hydrobromic acid, sulfuric acid, phosphoric acid, etc.). The acidic hydrolysis using trifluoroacetic acid is usually accelerated by adding a cation trapping agent (e.g. phenol, anisole, etc.).

The reaction is usually carried out in the presence of a conventional solvent that does not adversely affect the reaction, such as alcohol (e.g. methanol, ethanol, etc.), water, etc. However, dichloromethane, tetrahydrofuran, dioxane, acetone, etc., or a mixture thereof may also be used as a solvent. A liquid base or acid can also be used as a solvent.

The reaction temperature is not critical and the reaction is usually carried out under from cooling to heating.

### (ii) Reduction

The reduction method applicable for this elimination reaction may include, for example, reduction by using a combination of a metal (e.g. zinc, zinc amalgam, etc.) or a chrome compound (e.g. chromous chloride, chromous acetate, etc.) and an organic or inorganic acid (e.g. acetic acid, propionic acid, hydrochloric acid, sulfuric acid, etc.); and conventional catalytic reduction in the presence of a conventional metallic catalyst, such as palladium catalysts (e.g. spongy palladium, palladium black, palladium oxide, palladium on carbon, colloidal palladium, palladium on barium sulfate, palladium on barium carbonate, palladium hydroxide on carbon, etc.), nickel catalysts (e.g. reduced nickel, nickel oxide, Raney nickel, etc.), platinum catalysts (e.g. platinum plate, spongy platinum, platinum black, colloidal platinum, platinum oxide, platinum wire, etc.); sodium borohydride; lithium aluminum hydride; and the like.

The reaction is usually carried out in a conventional solvent that does not adversely influence the reaction, such as alcohol (e.g. methanol, ethanol, propanol, etc.), water, dioxane, tetrahydrofuran, acetic acid, buffer solution (e.g. phosphate buffer, acetate buffer, etc.), and the like, or a mixture thereof

The reaction temperature is not critical and the reaction is usually carried out under from cooling to heating.

The following Examples are given for the purpose of illustrating this invention in more detail. However, this invention are not limited to these.

### Preparation 1

N,O-bis(isopropoxycarbonyl)hydroxylamine (20.50 g), piperidine-1-ethanol (14.2 g) and triphenylphosphine (28.85 g) were dissolved in tetrahydrofuran (THF) (160 ml). A solution (40%, 48.0 g) of diethyl azodicarboxylate (DEAD) in toluene was added dropwise at an internal temperature of 20°C. After the addition was completed, reaction was carried out for 1 hour at room temperature. To the oil obtained by concentration, toluene (60 ml) and hexane (60 ml) were added, and the mixture was stirred for 2 hours under ice cooling to crystallize the reagent resolvent. The crystals were filtered and washed with toluene (20 ml) and hexane (20 ml). An object product was extracted twice (80 ml and 20 ml) using 2N hydrochloric acid, and the water layer was washed with diisopropyl ether (20 ml). Dichloromethane (100 ml) was added to the aqueous layer, and the pH of the solution was adjusted to 11 or more with aqueous caustic soda. The organic layer was separated and extracted by dichloromethane (20 ml). The combined dichloromethane layer was washed with saturated brine (40 ml) and concentrated under reduced pressure to obtain light orange oil (37.63 g). This was purified by a silica gel column (ethyl acetate-hexane = 1:1) to give N-(2-piperidinoethyl)-N,O-bis(isopropoxycarbonyl) hydroxylamine (28.4 g) as light yellow oil.
MS: 317 (M+1)
IR (film, cm⁻¹): 2983, 2937, 1789, 1739, 1720, 1384, 1238, 1108, 912
NMR (CDCl₃, δ): 1.27 (3H, d, J=6Hz), 1.35 (3H, d, J=6Hz), 1.3-1.7 (6H, m), 2.4 (4H, m), 2.56 (2H, t, J=7Hz), 3.76 (2H, t, J=7Hz), 4.9-5.1 (2H, m)

### Preparation 2

N,O-bis(tert-butoxycarbonyl)hydroxylamine (17.0 g), triphenylphosphine (17.68 g) and piperidine-1-ethanol (11.3 g) were dissolved in THF (170 ml) and ice-cooled. Diisopropyl azodicarboxylate (17.7 g) was added at an internal temperature of 10° or less. After the addition was completed, the reaction was continued for further 1 hour at room temperature.

After the reaction was completed, the reaction mixture was concentrated under reduced pressure, toluene (68 ml) was added thereto, and the mixture was concentrated once again. To the obtained oil, n-hexane (68 ml) was added and the mixture was stirred for 1.5 hours under ice cooling. The precipitated crystals were filtered and washed with n-hexane:toluene = 1:1 (34 ml). The filtrate was concentrated to give object crude oil of N-(2-piperidinoethyl)-N,O-bis(tert-butoxycarbonyl)hydroxylamine (31.2 g).
MS: 345 (M+1)
IR (film, cm⁻¹): 2979, 2935, 1785, 1716, 1369, 1128, 1043, 754
NMR (CDCl₃, δ): 1.48 (9H, s), 1.52 (9H, s), 1.3-1.7 (6H, m), 2.3-2.6 (4H, m), 2.56 (2H, t, J=7.4Hz), 3.6-3.9 (2H, m)

### Preparation 3

N-tert-butoxycarbonyl-O-benzylhydroxylamine (15.0 g), 1-(2-chloroethyl)piperidine hydrochloride (18.56 g) and potassium carbonate (27.81 g) were dissolved/suspended in dimethylformamide (75 ml) and the reaction was carried out for 9 hours at an internal temperature of 60°.

To the reaction solution, ethyl acetate (300 ml) was added, and the mixture was washed with water (150 ml). The ethyl acetate layer was further washed with water (75 ml) twice and washed with saturated brine. The organic layer was concentrated to give brown oil (21.4.g).

This was purified by silica gel column chromatography (ethyl acetate:n-hexane = 1:3) to give N-(2-piperidinoethyl)-N-tert-butoxycarbonyl-O-benzylhydroxylamine (13.74 g) as light yellow oil.
MS: 335 (M+1)
IR (film, cm⁻¹): 2935, 1702, 1456, 1390, 1367, 1168, 1132, 748, 698
NMR (CDCl₃, δ): 1.50 (9H, s), 1.3-1.7 (6H, m), 2.3-2.6 (4H, m), 2.51 (2H, t, J=7Hz), 3.55 (2H, t, J=7Hz), 4.85 (2H, s), 7.3-7.5 (5H, m)

### Preparation 4

N-(2-piperidinoethyl)-N-tert-butoxycarbonyl-O-benzylhydroxylamine (8.0 g) was dissolved in 4N hydrochloric acid-ethyl acetate solution (40 ml).

At room temperature, reaction was carried out for 4.5 hours, and the solvent was evaporated under reduced pressure to give N-(2-piperidinoethyl)-O-benzylhydroxylamine dihydrochloride (7.61 g) as white crystals.

The crystals were dissolved in water (40 ml) and dichloromethane (80 ml), and the pH of the mixture was adjusted to 10 or more with aqueous caustic soda. The aqueous layer was separated and further extracted by dichloromethane (20 ml), and the combined organic layer was washed with saturated brine (20 ml).

The organic layer was concentrated under reduced pressure to give colorless oil of N-(2-piperidinoethyl)-O-benzylhydroxylamine (5.58 g).
MS: 235 (M+1)
IR (film, cm⁻¹): 3029, 2935, 2854, 1454, 1301, 1155, 744, 698
NMR (CDCl₃, δ): 1.3-1.7 (6H, m), 2.3 (4H, m), 2.46 (2H, t, J=6Hz), 3.02 (2H, q, J=6Hz), 4.71 (2H, s), 5.94 (1H, t, J=6Hz), 7.2-7.5 (5H, m)

### Preparation 5

N-(2-piperidinoethyl)-O-benzylhydroxylamine (1.25 g) was dissolved in dichloromethane (12.5 ml), and pyridine (0.63 g) and acetic anhydride (0.81 g) were added thereto. After reaction was carried out for 3 hours at room temperature, the reaction mixture was washed with saturated sodium hydrogencarbonate, water and saturated brine.

The organic layer was concentrated under reduced pressure; furthermore, toluene was added thereto, and the mixture was concentrated under reduced pressure; this was repeated three times to give light yellow oil of N-acetyl-N-(2-piperidinoethyl)-O-benzylhydroxylamine (1.34 g).
MS: 277 (M+1)
IR (film, cm⁻¹): 3031, 2935, 2854, 1666, 1398, 1211, 1157, 910, 754, 698
NMR (CDCl₃, δ): 1.3-1.7 (6H, m), 2.07 (3H, s), 2.3-2.5 (4H, m), 2.27 (2H, t, J=8.5Hz), 3.77 (2H, m), 4.87 (2H, s), 7.38 (5H, s)

### Example 1-1)

Crude oil (31.2 g) of N-(2-piperidinoethyl)-N,O-bis(tert-butoxycarbonyl)hydroxylamine was dissolved in 4N hydrochloric acid-ethyl acetate solution (170 ml), and the mixture was stirred at room temperature. The product was separated in the middle of the reaction and then crystallized. After strirring for 3 hours at room temperature, the precipitated crystals were filtered. The obtained crystals were dried under reduced pressure to give N-(2-piperidinoethyl)
hydroxylamine dihydrochloride as light yellow crystals.
NMR (CDCl₃, δ): 1.4-2.1 (6H, m), 2.9-3.7 (4H, m), 3.59 (2H, t, J=6.4Hz), 3.80 (2H, t, J=6.4Hz)

### Example 1-2)

N-(2-piperidinoethyl)-N,O-bis(isopropoxycarbonyl) hydroxylamine (25.0 g) was dissolved in concentrated hydrochloric acid (160 ml) and heated in an oil bath to reflux for 3 hours.

After concentration under reduced pressure, isopropyl alcohol and toluene were added to the residue, and the mixture was concentrated under reduced pressure repeatedly to remove water and give brown hygroscopic crystals.

The crystals were dried by a vacuum pump to give N-(2-piperidinoethyl)hydroxylamine dihydrochloride (18.5 g) as brown hygroscopic crystals.
NMR (CD₃OD, δ): 1.5-2.1 (6H, m), 2.9-3.3 (4H, m), 3.63 (2H, t, J=6.4Hz), 3.84 (2H, t, J=6.4Hz)

### Example 2

N-(2-piperidinoethyl)hydroxylamine dihydrochloride (15.0 g) was dissolved in water (60 ml) and THF (60 ml), and cooled in an ice bath.

While the pH of the mixture was adjusted to 8.3 - 9.2 with 24% aqueous caustic soda at an internal temperature of 0 - 10°C, benzoic chloride (19.5 g) was added over approximately 60 minutes.

After the addition was completed, the mixture was stirred continuously for further 30 minutes at the same temperature and the same pH.

THF (60 ml) was added to the reaction solution to carry out extraction, and the reaction solution was further extracted with THF (20 ml). The combined THF layer was washed with saturated brine (30 ml) and concentrated under reduced pressure to give N-(2-piperidinoethyl)-N,O-dibenzylhydroxylamine (23.9 g) as brown crystals.

The crystals were dissolved in methanol (60 ml) and stirred for 4 hours at room temperature. The reaction solution was concentrated under reduced pressure, toluene was added thereto, and the mixture was concentrated again to give crude crystals of N-(2-piperidinoethyl)-N-benzoylhydroxylamine (hereafter referred to as compound (Ia)). After toluene (30 ml) was added thereto, the mixture was suspended and crystallized, and then filtered and dried to give compound (Ia) (14.6 g) as light brown crystals.
mp: 108-109°C
MS: 249 (M+1)
IR (nujol, cm⁻¹): 2929, 2854, 1619, 1376, 1216, 1170, 925, 787, 700
NMR (CD₃OD, δ): 1.3-1.8 (6H, m), 2.3-2.7 (4H, m), 2.71 (2H, t, J=7Hz), 3.8 (2H, m), 7.3-7.6 (3H, m), 7.6-7.8 (2H, m)

### Example 3

N-(2-piperidinoethyl)-N,O-bis(isopropoxycarbonyl) hydroxylamine (40.0 g) was dissolved in methanol (200 ml), and 30% methylamine-ethanol solution (4.55 g) was added thereto at room temperature. After stirring for 1 hour at room temperature, the mixture was concentrated and dried. To the residue, n-heptane (100 ml) was added, and the mixture was stirred for 2 hours under ice cooling to crystallize the object product. The crystals were filtered and washed with n-heptane (40 ml), and then dried under vacuum to give white crystals of N-(2-piperidinoethyl)-N-(isopropoxycarbonyl) hydroxylamine (hereafter referred to as compound (Ib)) (26.4 g).
mp: 78-79°C
MS: 231 (M+1)
IR (nujol, cm⁻¹): 2925, 1683, 1457, 1438, 1375, 1211, 1112, 1020, 792
NMR (CDCl₃, δ): 1.26 (6H, d, J=6Hz), 1.4-1.8 (6H, m), 2.5 (4H, m), 2.66 (2H, t, J=5Hz), 3.70 (2H, t, J=5Hz), 4.96 (1H, sep, J=6Hz)

### Example 4

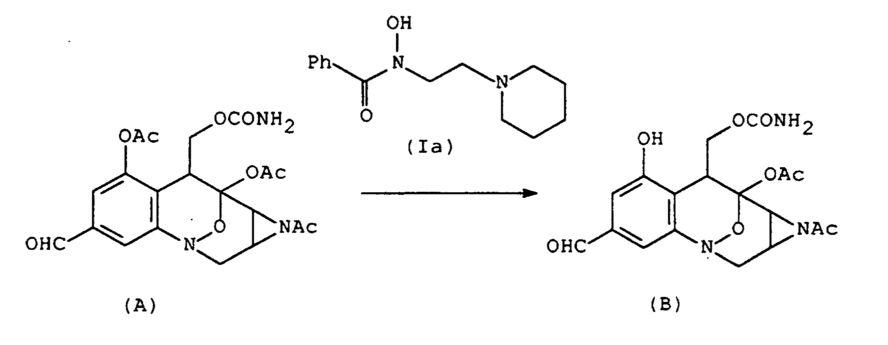

in which Ph is phenyl.

After the compound (A) (7 g) and the compound (Ia) (0.388 g) were dissolved in THF (56 ml) and methanol (MeOH) (14 ml) and heated to 45°C, reaction was carried out for 7 hours and 30 minutes at the same temperature.

To the reaction solution, ethyl acetate (70 ml) and 10% brine (140 ml) were added, whereby extraction was carried out. The separated aqueous layer was re-extracted with ethyl acetate (35 ml), the organic layers were combined, and the combined organic layer was washed with 10% brine (70 ml).

The separated brine layer was re-extracted with ethyl acetate (35 ml), the organic layers were combined, and the combined organic layer was washed with saturated brine (140 ml).

The organic layer was concentrated (crystallized), methylene chloride (70 ml) was added thereto, whereby crystallization was carried out.

After the solution including the crystals was allowed to stand overnight at 10°C or less, the crystals were filtered and dried overnight under vacuum to give compound (B) (6.2 g) (97.7%) as light yellow crystals.
NMR (DMSO-d₆, δ): 10.4 (1H, br s), 9.80 (1H, s), 6.97 (1H, br s), 6.87 (1H, br s), 4.25 (1H, dd, J=8.3, 9.6Hz), 3.95 (1H, br d, J=9.6Hz), 3.7-3.9 (3H, m), 3.64 (1H, d, J=6.3Hz), 2.89 (1H, br d, J=6.3Hz), 2.16 (3H, s), 1.83 (3H, s)

### Example 5

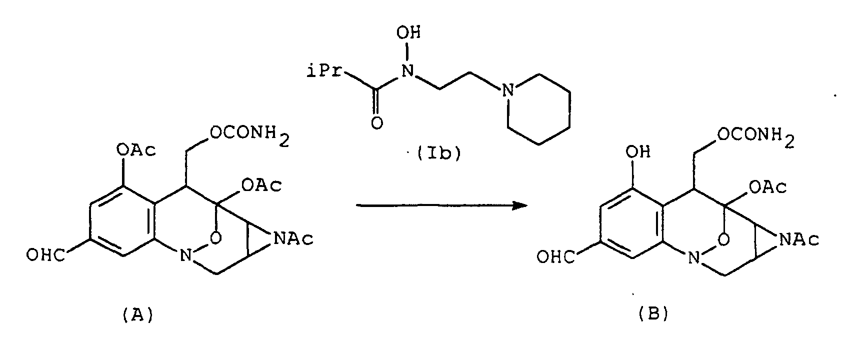

After the compound (A) (7 g) and the compound (Ib) (0.72 g) were dissolved in THF (56 ml) and methanol (MeOH) (14 ml) and heated to 45°C, reaction was carried out for 22 hours at the same temperature under stirring.

To the reaction solution, ethyl acetate (70 ml) and 10% brine (140 ml) were added, whereby extraction was carried out, and the separated aqueous layer was re-extracted with ethyl acetate (35 ml). After the organic layers were combined, the combined organic layer was washed with 10% brine (70 ml). The separated brine layer was re-extracted with ethyl acetate (35 ml). After the organic layers were combined, the combined organic layer was washed with saturated brine (140 ml).

The organic layer was concentrated (crystallized), methylene chloride (70 ml) was added thereto, and the mixture was stirred, whereby crystallization was carried out.

After the solution including the crystals was allowed to stand overnight at 10°C or less, the crystals were filtered and dried (40°C) under vacuum to give compound (B) (5.7 g) (89.9%) as light yellow crystals.
NMR (DMSO-d₆, δ): 10.4 (1H, br s), 9.80 (1H, s), 6.96 (1H, br s), 6.87 (1H, br s), 4.25 (1H, dd, J=8.3, 9.6Hz), 3.95 (1H, br d, J=9.6Hz), 3.7-3.9 (3H, m), 3.64 (1H, d, J=6.3Hz), 2.89 (1H, br d, J=6.3Hz), 2.16 (3H, s), 1.83 (3H, s)

### Example 6

1,5-diacetoxy-1,2,3,4-tetrahydronaphthalene (2.5 g) and the compound (Ia) (0.5 g) were dissolved in THF (20 ml) and MeOH (5 ml), and reaction was carried out for 7.5 hours at 40°C. After the reaction was completed, the reaction mixture was concentrated under reduced pressure, ethyl acetate (50 ml) was added to the residue so that the residue was dissolved, and the solution was washed with 1N hydrochloric acid, water and saturated brine.

The ethyl acetate layer was concentrated to give crude 1-acetoxy-5-hydroxy-1,2,3,4-tetrahydronaphthalene (2.17 g).

This was recrystalized from a small amount of ethanol to give white crystals of 1-acetoxy-5-hydroxy-1,2,3,4-tetrahydronaphthalene (1.7 g).
NMR (CDCl₃, δ): 1.8-2.0 (4H, m), 2.09 (3H, s), 2.4-2.7 (1H, m), 2.7-3.0 (1H, m), 5.9 (1H, m), 6.7 (1H, d, J=8Hz), 6.9 (1H, d, J=8Hz), 7.1 (1H, t, J=8Hz)

### Example 7

1,5-diacetoxy-1,2,3,4-tetrahydronaphthalene (2.48 g) and the compound (Ib) (2.53 g) were dissolved in THF (19.8 ml) and MeOH (5 ml), and reaction was carried out for 7.5 hours at 40°C. After the reaction was completed, the reaction mixture was concentrated under reduced pressure, ethyl acetate (50 ml) was added to the residue so that the residue was dissolved, and the solution was washed with 1N hydrochloric acid, water and saturated brine.

The ethyl acetate layer was concentrated to give crude 1-acetoxy-5-hydroxy-1,2,3,4-tetrahydronaphthalene (2.31 g).

This was recrystalized from ethanol to give white crystals of 1-acetoxy-5-hydroxy-1,2,3,4-tetrahydronaphthalene (1.93 g).
NMR (CDCl₃, δ): 1.8-2.0 (4H, m), 2.09 (3H, s), 2.4-2.7 (1H, m), 2.7-3.0 (1H, m), 5.9 (1H, m), 6.7 (1H, d, J=8Hz), 6.9 (1H, d, J=8Hz), 7.1 (1H, t, J=8Hz)

### Example 8

Ethyl 4-acetoxybenzoate (3.6 g) was dissolved in methanol (28.8 ml) and THF (7.2 ml).

The compound (Ia) (0.46 g) was added to the solution, and the mixture was stirred for 30 minutes at 40°C.

The reaction solution was concentrated under reduced pressure and crystallized with water.

The crystals were collected by filtration and dried to give ethyl 4-hydroxybenzoate (2.68 g) as white crystals.
NMR (CDCl₃, δ): 1.38 (3H, t, J=7.1Hz), 4.36 (2H, q, J=7.1Hz), 6.90 (2H, d, J=9Hz), 7.95 (2H, d, J=9Hz)

### Example 9

Ethyl 4-acetoxybenzoate (2.0 g) and the compound (Ib) (2.85 g) were dissolved in methanol (2 ml) and THF (16 ml). The mixture was stirred for 1 hour at 40°C. After the reaction was completed, the reaction mixture was concentrated under reduced pressure, ethyl acetate (50 ml) was added to the residue so that the residue was dissolved, and the solution was washed with 1N hydrochloric acid, water and saturated brine. The ethyl acetate layer was concentrated to give crude crystals (1.7 g) of ethyl 4-hydroxybenzoate.

The crude crystals were suspended in water and purified to give ethyl 4-hydroxybenzoate (1.56 g).
NMR (CDCl₃, δ): 1.38 (3H, t, J=7.1Hz), 4.36 (2H, q, J=7.1Hz), 6.90 (2H, d, J=9Hz), 7.95 (2H, d, J=9Hz)

### Example 10

N-(2-piperidinoethyl)-N-tert-butoxycarbonyl-O-benzylhydroxylamine (1.0 g) was dissolved in anhydrous ethanol (20 ml).

10%-Pd/C (0.2 g) was added thereto, and reaction was carried out for 2 hours at a hydrogen pressure of 2 kg/cm².

After the catalyst was filtered off, the solution was concentrated under reduced pressure to give N-(2-piperidinoethyl)-N-tert-butoxycarbonylhydroxylamine (0.7 g) as light yellow oil.
MS: 245 (M+1)
IR (film, cm⁻¹): 2935, 2856, 1695, 1444, 1365, 1251, 1133, 1041, 758
NMR (CDCl₃, δ): 1.53 (9H, s), 1.5-1.8 (6H, m), 2.3-2.7 (4H, m), 2.74 (2H, t, J=5.5Hz), 3.71 (2H, t, J=5.5Hz)

### Example 11

N-acetyl-N-(2-piperidinoethyl)-O-benzylhydroxylamine (1.0 g) was dissolved in anhydrous ethanol (50 ml), and 10%-Pd/C (0.2 g) was added thereto. Reaction was carried out for 1.5 hours at a hydrogen pressure of 2 kg/cm².

After the reaction was completed, the catalyst was filtered off, and the solution was concentrated under reduced pressure to give N-acetyl-N-(2-piperidinoethyl)hydroxylamine (0.68 g) as almost colorless oil.
MS: 187 (M+1)
IR (film, cm⁻¹) : 2935, 2856, 1635, 1456, 1214, 1128, 1039, 754
NMR (CDCl₃, δ): 1.3-1.8 (6H, m), 2.13 (6H, s), 2.3-2.7 (4H, m), 2.67 (2H, t, J=5.5Hz), 3.85 (2H, t, J=5.5Hz)

### Example 12

The compound A (3 g) and the compound Ia (0.08 g) were suspended in 80% aqueous methanol (15 ml), and reaction was carried out for 4 hours at an internal temperature of 45°. After the reaction was completed, water (45 ml) was added thereto at room temperature, and the compound B was crystallized. Then, mixture was stirred overnight under ice cooling and filtered the next day. The crystals were washed with 20% aqueous methanol and dried under vacuum at 40° to give compound B (2.51 g) (92.3%) as white crystals.

### Example 13

The compound A (7 g), N,N-dimethyl-1,3-propanediamine (compound Ik) (3.2 g) were dissolved in THF (56 ml) and methanol (14 ml) and stirred at an internal temperature of 45°. After 4 hours, N,N-dimethyl-1,3-propanediamine (0.16 g) was added thereto, and reaction was carried out for further 1 hour. To the reaction liquid, ethyl acetate (70 ml) and 10% brine (140 ml) were added to extract the object product. The aqueous layer was extracted with ethyl acetate (35 ml), the combined ethyl acetate layer was washed with saturated brine and then concentrated. The obtained oil was pulverized with n-heptane, filtered and dried to give compound B (4.0 g) (63.1%).

The following compounds were obtained in a similar manner to that of Example 3.

### Example 14

N-(2- (dimethylamino)ethyl) -N- (isopropoxycarbonyl) hydroxylamine
MS: 191 (M+1)
IR (film, cm⁻¹): 2979, 1697, 1255, 1180, 1106, 925, 758
NMR (CDCl₃, δ): 1.29 (6H, d, J=6.3Hz), 2.30 (6H, s), 2.61 (2H, t, J=5.8Hz), 3.68 (2H, t, J=5.8Hz), 4.94 (1H, sep, J=6.3Hz)

### Example 15

N-(3-(dimethylamino)propyl)-N-(isopropoxycarbonyl) hydroxylamine
MS: 205 (M+1)
IR (film, cm⁻¹) : 2979, 1716, 1375, 1259, 1238, 1106, 923, 757
NMR (CDCl₃, δ); 1.29 (6H, d, J=6.2Hz), 1.80 (2H, m), 2.28 (2H, s), 2.44 (2H, t, J=6Hz), 3.69 (2H, t, J=6Hz), 4.97 (1H, sep, J=6.2Hz)

### Example 16

N-propionyl-N-(2-(piperidinoethyl)hydroxylamine was obtained in a similar manner to that of Example 11.
IR (film, cm⁻¹): 2940, 2877, 1641, 1633, 1290, 1240, 1203, 1091, 754
NMR (CDCl₃, δ): 1.13 (6H, t, J=7.4Hz), 1.6 (2H, m), 1.8 (4H, m), 2.50 (2H, q, J=7.4Hz), 3.0 (4H, m), 3.06 (2H, t, J=5.3Hz), 3.98 (2H, t, J=5.3Hz)

## Claims

1. A selective deacetylating method using a compound of the formula (I): 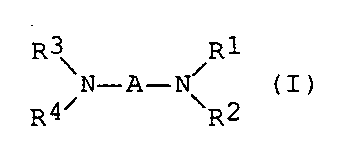 in which R¹ and R² are each lower alkyl or combined together to form lower alkylene, R³ is hydrogen or hydroxy, R⁴ is hydrogen or acyl, and A is lower alkylene.

2. A selective deacetylating method of claim 1, wherein R¹ and R² are each lower alkyl or combined together to form lower alkylene, R³ is hydroxy, and R⁴ is acyl.

3. A selective deacetylating method of claim 2, wherein R¹ and R² are combined together to form lower alkylene, and R⁴ is lower alkanoyl, lower alkoxycarbonyl or benzoyl.

4. A selective deacetylating method of claim 1, wherein R¹ and R² are each lower alkyl, and R³ and R⁴ are each hydrogen.

5. A process for preparing a compound of the formula (B): 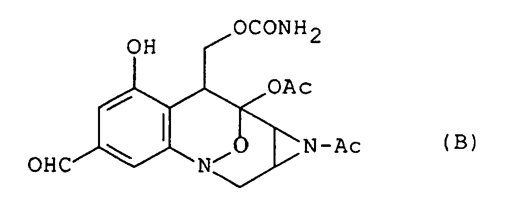 wherein Ac is acetyl,
which comprises,
reacting a compound of the formula (A): 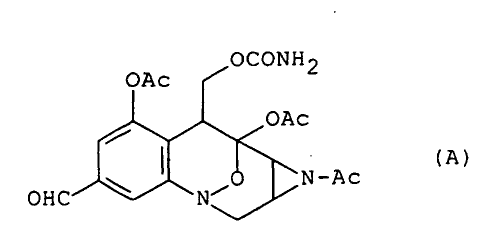 wherein Ac is acetyl,
with a compound of the formula (I): 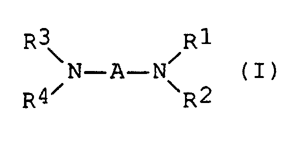 in which R¹ and R² are each lower alkyl or combined together to form lower alkylene, R³ is hydrogen or hydroxy, R⁴ is hydrogen or acyl, and A is lower alkylene.

6. A preparation process of claim 5, wherein R¹ and R² are each lower alkyl or combined together to form lower alkylene, R³ is hydroxy, and R⁴ is acyl.

7. A preparation process of claim 6, wherein R¹ and R² are combined together to form lower alkylene, and R⁴ is lower alkanoyl, lower alkoxycarbonyl or benzoyl.

8. A preparation process of claim 6, wherein R¹ and R² are each lower alkyl, and R³ and R⁴ are each hydrogen.

9. A compound of the formula (I-a): 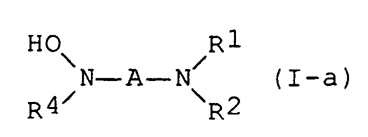 in which R¹ and R² are each lower alkyl or combined together to form lower alkylene, R⁴ is acyl, and A is lower alkylene; however, when R¹ and R² are each lower alkyl, R⁴ is acyl other than lower alkanoyl, or a salt thereof.

10. A compound of claim 9, wherein R¹ and R² are each lower alkyl or combined together to form lower alkylene, and R⁴ is acyl.

11. A compound of claim 10, wherein R¹ and R² are combined together to form lower alkylene, and R⁴ is lower alkanoyl, lower alkoxycarbonyl or benzoyl.
